# EUROPEAN PATENT SPECIFICATION

(11) **EP 3 831 321 B1**
(45) Date of publication and mention of the grant of the patent: **25.01.2023**
(21) Application number: 20211882.4
(22) Date of filing: 04.12.2020
(51) Int. Cl.: A61B 17/34

(54) **SEAL ASSEMBLIES FOR SURGICAL ACCESS ASSEMBLIES**
DICHTUNGSANORDNUNGEN FÜR CHIRURGISCHE ZUGANGSANORDNUNGEN
ENSEMBLES JOINTS D'ÉTANCHÉITÉ POUR ENSEMBLES D'ACCÈS CHIRURGICAL

(30) Priority: 04.12.2019 US 201916703383
(43) Date of publication of application: 09.06.2021
(73) Proprietor: Covidien LP, Mansfield, MA 02048 (US)
(72) Inventor: PILLETERE, Roy J., North Haven, CT 06473 (US); DININO, Matthew A., Newington, CT 06111 (US); EBERSOLE, Garrett, Hamden, CT 06517 (US); DESJARDIN, Kevin, Prospect, CT 06712 (US); THOMAS, Justin, New Haven, CT 06512 (US); BARIL, Jacob C., Norwalk, CT 06851 (US); LAPIERRE (Roy), Nicolette, Windsor Locks, CT 06096 (US); BROWN, Eric, Madison, CT 06443 (US)
(74) Representative: Maschio, Antonio

(56) References cited:
- EP-A1- 3 449 853
- WO-A1-2012/131746
- WO-A1-2016/186905
- WO-A1-2018/077226
- US-A1- 2005 077 689
- US-A1- 2018 021 063
- US-A1- 2019 350 619
- US-B2- 8 197 404

## Description

### CROSS-REFERENCE TO RELATED APPLICATIONS

This application is a continuation-in-part application of U.S. Patent Application Serial No. 16/272,068, filed February 11, 2019.

### FIELD

The present disclosure relates to access assemblies for minimally invasive surgery, including seals. More particularly, the present disclosure relates to seals for surgical access assemblies.

### BACKGROUND

In order to facilitate minimally invasive surgery, a working space must be created at the desired surgical site. An insufflation gas, typically CO₂, is introduced into the abdomen of the patient to create an inflated state called a pneumoperitoneum. Access assemblies are utilized to allow the introduction of surgical instrumentation and endoscopes (or other visualization tools). These access assemblies maintain the pressure for the pneumoperitoneum, as they have one or more seals that adapt to the surgical instrumentation. Typically, a "zero-seal" in the access assembly seals the access assembly in the absence of a surgical instrument in the access assembly, and an instrument seal seals around a surgical instrument that has been inserted through the access assembly.

The breadth of surgical instrumentation on the market today requires a robust seal capable of adjusting to multiple sizes and withstanding multiple insertions of surgical instrumentation. Some of the instrumentation can include sharp edges that can tear or otherwise damage seals. Therefore, it would be beneficial to have an access assembly with improved seal durability. US 2019/350619 A1 and WO 2018/077226 A1 describe surgical access assemblies according to the preamble of claim 1, and including a plurality of overlapping guard sections. WO 2012/131746 A1 describes a surgical access assembly with a guard assembly formed by a plurality of guard sections and a seal assembly.

### SUMMARY

An access assembly with an improved seal durability is provided. The access assembly includes an instrument valve housing and a valve assembly. The instrument valve housing defines a cavity. The valve assembly is disposed within the cavity of the instrument valve housing. The valve assembly includes a guard assembly, a seal assembly disposed adjacent to the guard assembly, and a centering mechanism for maintaining the seal assembly and guard assembly centered within the cavity of the instrument valve. The guard assembly includes a support ring and a plurality of guard sections disposed distally of the support ring. The seal assembly includes a proximal seal member, an intermediate seal assembly, and a distal seal member.

In embodiments, the guard assembly includes six guard sections. Each guard section of the plurality of guard sections may include a first side configured to accommodate an overlapping portion of an adjacent guard section of the plurality of guard sections. The first side of each guard section of the plurality of guard sections may angle downwardly. Each guard section of the plurality of guard sections may include a guard portion having a substantially kidney shape. Each guard portion may include an indentation and may be configured such that when the plurality of guard sections are assembled, the indentations form an opening.

In some embodiments, at least one of the support ring and the plurality of guard sections includes a plurality of outward extending tabs for aligning the guard assembly with the centering mechanism. The proximal seal member may include an annular flange and a seal portion supported within the annular flange. The intermediate seal assembly includes a plurality of seal sections. The access assembly may further include a retaining member having a plurality of pins. The centering mechanism may include an annular base defining a channel for receiving the plurality of pins for maintaining the valve assembly in an assembled configuration.

Another access assembly with an improved seal durability includes an instrument valve housing and a valve assembly disposed within the instrument valve housing. More particularly, the instrument valve housing includes upper, lower, and inner housing sections and defines a cavity in which the valve assembly is received. The valve assembly includes a guard assembly, a seal assembly disposed adjacent to the guard assembly, and a centering mechanism for maintaining the seal assembly and guard assembly centered within the cavity of the instrument valve. The guard assembly includes a plurality of guard sections. The seal assembly includes a proximal seal member, an intermediate seal assembly, and a distal seal member. The intermediate seal assembly includes a plurality of seal sections in a stacked configuration; and

In embodiments, the intermediate seal assembly includes six seal sections. Each seal section of the plurality of seal sections may include a wing shape. An inner edge of each seal section of the plurality of seal sections may be straight. Alternatively, the inner portion of each seal section of the plurality of seal sections is tapered. At least one of a top surface and a bottom surface of the inner portions may taper. The intermediate seal assembly may be hexagonal. The proximal seal member may include an annular flange and a seal portion supported within the annular flange. The proximal and distal seal members may operate to support the intermediate seal assembly. The access assembly may further include a retainer assembly including upper and lower retainer members. At least one of the upper and lower retainer members may include a plurality of pins receivable through the guard assembly and seal assembly for retaining the guard and seal assemblies relative to each other.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiments given below, serve to explain the principles of the disclosure, wherein:
FIG. 1 is a side perspective view of an access assembly according to an embodiment of the present disclosure;
FIG. 2 a side cross-sectional view of a proximal region of the access assembly shown in FIG. 1 taken along section line 2-2;
FIG. 3 is an exploded perspective view of a valve assembly, including a centering mechanism, a guard assembly, a seal assembly, and a retainer assembly;
FIG. 4 is a perspective view of the seal assembly shown in FIG. 3;
FIG. 5 is a perspective view of the seal assembly shown in FIG. 3, in an unfolded configuration;
FIG. 6-11 are perspective views of the seal assembly shown in FIG. 3, in sequential partially folded and fully folded conditions;

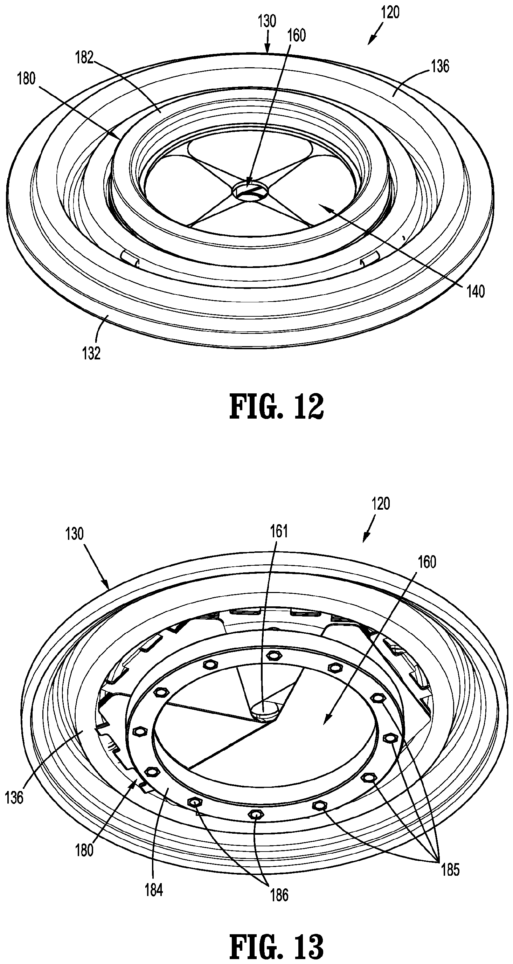

FIG. 12 is a top perspective view of the valve assembly shown in FIG. 3;
FIG. 13 is a bottom perspective view of the valve assembly shown in FIG. 3;
FIG. 14 is a top view of a seal assembly according to another example of the present disclosure, in an initial or unfolded configuration;
FIG. 15 is a perspective side view of a section of the seal assembly shown in FIG. 14;
FIG. 16 is a side cross-sectional view of a section the seal assembly shown in FIG. 14 taken along section line 16-16 shown in FIG. 15;
FIG. 17 is a top view of the seal assembly shown in FIG. 14, in a folded configuration;
FIG. 18 is a perspective view of a valve assembly according to the invention;
FIG. 19 is an exploded perspective view of the valve assembly shown in FIG. 18, including a centering mechanism, a guard assembly, a seal assembly, and a retainer assembly;
FIG. 20 is a top perspective view of the centering mechanism shown in FIG. 19;
FIG. 21 is a side cross-sectional view of the centering mechanism shown in FIG. 20, taken along line 21-21;
FIG. 22 is a top view of a guard section of the guard assembly shown in FIG. 19;
FIG. 23 is a cross-sectional side view of the guard section shown in FIG. 20, taken along line 23-23;
FIGS. 24-29 are perspective views of the guard assembly shown in FIG. 19, in sequential order of assembly;
FIG. 30 is a side cross-sectional view of the valve assembly shown in FIG. 18;
FIG. 31 is a perspective view of a valve assembly according to yet another example of the present disclosure;
FIG. 32 is an exploded perspective view of the valve assembly shown in FIG. 31, including a centering mechanism, a guard assembly, a seal assembly, and a retainer assembly;
FIG. 33 is a top perspective view of seal sections of the seal assembly shown in FIG. 32;
FIG. 34 is a side view of the seal sections shown in FIG. 33;
FIG. 35 is a top view of a seal section of the seal assembly shown in FIG. 33;
FIG. 36 is a side cross-sectional view of the seal section shown in FIG. 35, taken along line 36-36; and
FIGS. 37-42 are perspective views of the seal sections shown in FIG. 33, in sequential order of assembly.

### DETAILED DESCRIPTION

Particular embodiments of the present disclosure are described hereinbelow with reference to the accompanying drawings; however, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure. Like reference numerals refer to similar or identical elements throughout the description of the figures.

As used herein, the term "distal" refers to that portion of the instrument, or component thereof which is farther from the user while the term "proximal" refers to that portion of the instrument or component thereof which is closer to the user.

Access assemblies with obturators are employed during minimally invasive surgery, e.g., laparoscopic surgery, and provide for the sealed access of surgical instruments into an insufflated body cavity, such as the abdominal cavity. The access assemblies of the present disclosure include an instrument valve housing mounted on a cannula tube, and include an obturator (not shown) inserted through the valve housing and cannula. The obturator can have a blunt distal end, or a bladed or non-bladed penetrating distal end and can be used to incise the abdominal wall so that the access assembly can be introduced into the abdomen. The handle of the obturator can engage or selectively lock into the instrument valve housing of the access assembly.

Access assemblies are employed to tunnel through an anatomical structure, e.g., the abdominal wall, either by making a new passage through the anatomical structure or by passing through an existing opening through the anatomical structure. Once the trocar assembly with the obturator has tunneled through the anatomical structure, the obturator is removed, leaving the access assembly in place. The instrument valve housing of the access assembly includes valves that prevent the escape of insufflation gases from the body cavity, while also allowing surgical instruments to be inserted into the cavity.

In various examples, a bladeless optical trocar obturator may be provided that permits separation of tissue planes in a surgical procedure and visualization of body tissue fibers as they are being separated, thereby permitting a controlled traversal across a body wall. In other examples, the trocar obturator may be bladeless without being optical, e.g., without providing contemporaneous visualization thereof through the distal tip of an obturator. The bladeless obturator may be provided for the blunt dissection of the abdominal lining during a surgical procedure.

Various trocar obturators suitable for use with the access assembly of the present disclosure are known and include, for example, bladed, bladeless, blunt, optical, and non-optical. For a detailed description of the structure and function of exemplary trocar assemblies, including exemplar trocar obturators and exemplar cannulas, please refer to commonly owned PCT Publication No. WO 2016/186905 ("the '905 publication").

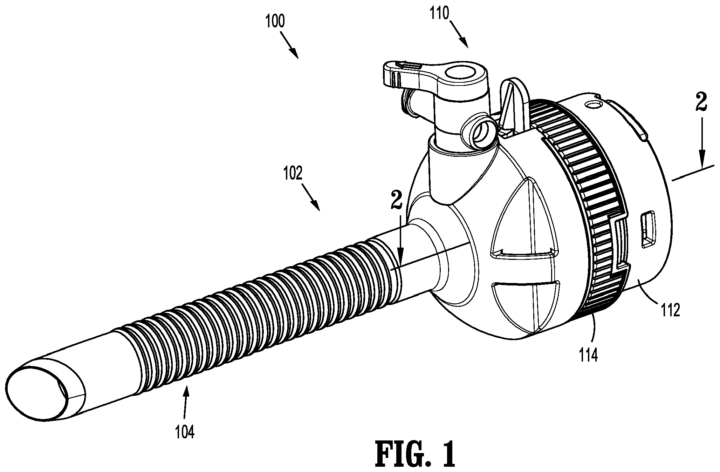

With initial reference now to FIG. 1, an access assembly according to aspects of the present disclosure is shown generally as access assembly 100. The access assembly 100 includes a cannula 102 and an instrument valve housing 110 secured to the cannula 102. For a detailed description of an exemplary access assembly, please refer to the '905 publication.

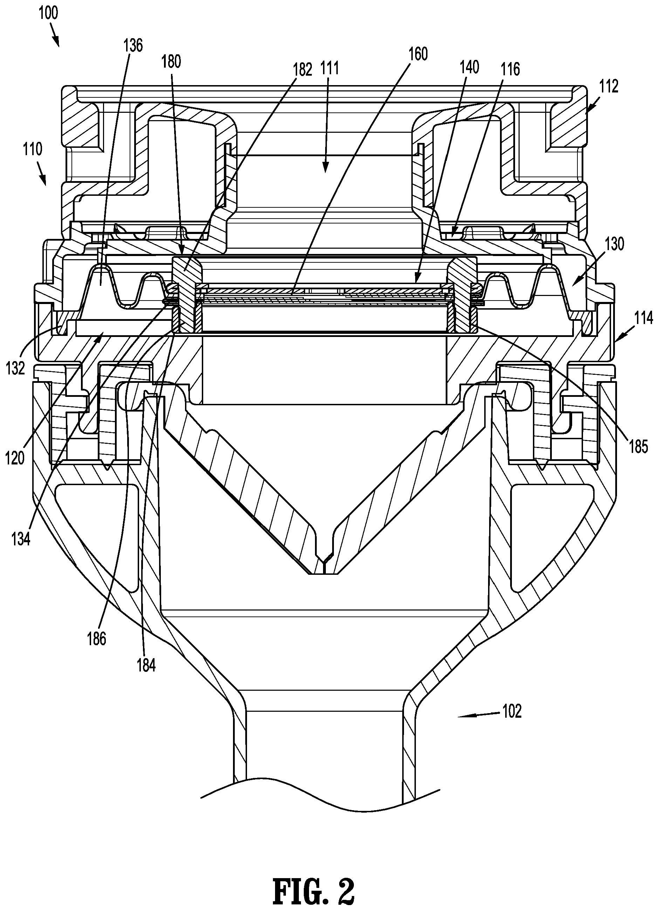

With reference to FIG. 2, the instrument valve housing 110 of the access assembly 100 includes an upper housing section 112, a lower housing section 114, and an inner housing section 116. The upper, lower, and inner housing sections 112, 114, 116 are configured to support a valve assembly 120 on a proximal end of the cannula 102. More particularly, the inner housing section 116 is secured between the upper and lower housing sections 112, 114, and the valve assembly 120 is received between the inner and lower housing sections 116, 114. The upper and lower housing sections 112, 114 of the instrument valve housing 110 may be selectively attachable to, and detachable from, the inner housing section 116. The lower housing section 114 may be releasably or permanently attached to a cannula tube 104 (FIG. 1) of the access assembly 102. In examples, either or both of the upper and lower housing sections 112, 114 of the instrument valve housing 110 may include knurls, indentations, tabs, or be otherwise configured to facilitate engagement by a clinician.

The access assembly 100 may also include features for the stabilization of the access assembly 100. For example, the distal end of the cannula tube 104 may carry a balloon anchor or another expandable member that engages the abdomen from the interior side. For example, see commonly owned U.S. Pat. No. 7,300,448. A feature on the opposite side of the abdominal wall may be used to further stabilize the access assembly, such as adhesive tabs or adjustable foam collars.

The upper, lower, and inner housing sections 112, 114, 116 of the instrument valve housing 110 define a longitudinal passage 111 for receipt of a surgical instrument (not shown). The valve assembly 120 is supported within the instrument valve housing 110 to provide sealed passage of the surgical instrument through the access assembly 100.

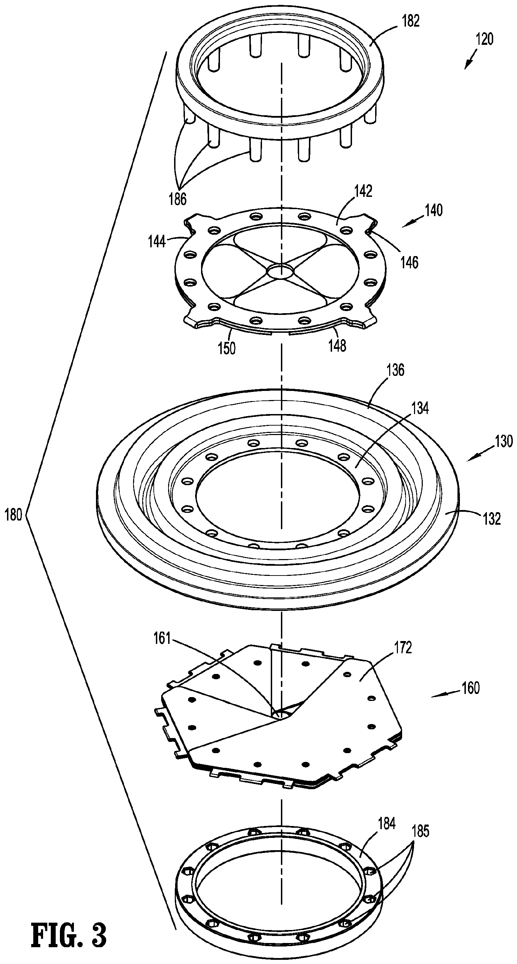

With particular reference to FIGS. 2 and 3, the valve assembly 120 supported in the instrument valve housing 110 (FIG. 2) includes a centering mechanism 130, a guard assembly 140, a seal assembly 160, and a retainer assembly 180. The centering mechanism 130 of the valve assembly 120 permits radial movement of the valve assembly 120 relative to the instrument valve housing 110 when a surgical instrument is received through the valve assembly 120, and returns the valve assembly 120 to a generally centered position once the surgical instrument is withdrawn from within the instrument valve housing 110. The guard assembly 140 protects the seal assembly 160 during insertion and withdrawal of a surgical instrument through the seal assembly 160. The seal assembly 160 provides sealed passage of the surgical instrument through the instrument valve housing 110. The retainer assembly 180 maintains the centering mechanism 130, the guard assembly 140, and the seal assembly 160 in an aligned relationship with one another.

With continued reference to FIGS. 2 and 3, as noted above, the centering mechanism 130 of the valve assembly 120 is configured to maintain the valve assembly 120 centered within the instrument valve housing 110 (FIG. 2). In examples, and as shown, the centering mechanism 130 includes an outer annular ring 132, an inner annular ring 134, and a bellows 136 disposed between the outer annular ring 132 and the inner annular ring 134. As shown in FIG. 2, the outer annular ring 132 is received between the inner housing section 116 and the lower housing section 114 to retain the centering mechanism 130 within the instrument valve housing 110. The inner annular ring 134 supports the seal assembly 160. For a detailed description of the structure and function of an exemplary centering mechanism, please refer to commonly owned U.S. Pat. No. 6,702,787 ("the '787 patent").

Although shown including the centering mechanism 130 having bellows 136, the valve assembly 120 may include alternative centering mechanisms. For example, the centering mechanism may include an annular base and a plurality of spokes extending from the base, as described in commonly owned U.S. Pat. App. Pub. No. 2015/0025477 ("the '477 publication"). It is envisioned that the centering mechanism may include multiple sets of spokes, as disclosed in the '477 publication.

Still referring to FIGS. 2 and 3, the guard assembly 140 of the valve assembly 120 includes a ring portion 142 and first, second, third, and fourth petals 144, 146, 148, 150. The guard assembly 140 may be formed from a sheet of plastic/polymeric material by stamping with a tool that forms the ring portion 142 and the petals 144, 146, 148, 150. Alternatively, the guard assembly 140 may be formed by molding or other techniques. It is envisioned that the guard assembly may include any number of petals, and the petals may include flap portions of any size or configuration. See, for example, U.S. Patent Nos. 5,895,377 and 6,569,120 ("the '377 and '120 Patents"), and PCT Publication WO 91/12838, for exemplary guard assemblies, as well as other aspects of access assemblies. For a detailed description of the structure and function of exemplary guard assemblies, please refer to commonly owned U.S. Pat. App. Ser. Nos. 16/394,043 and 16/238,823.

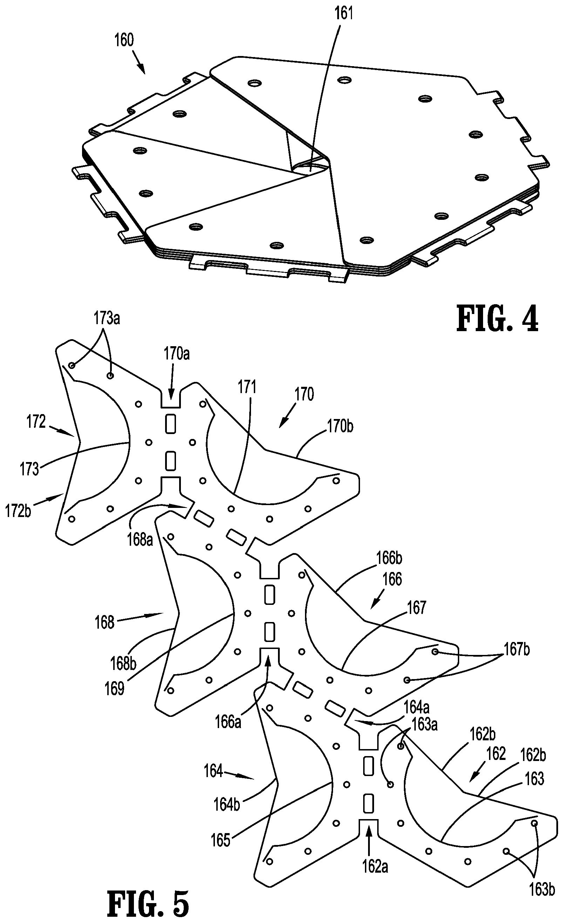

With particular reference now to FIGS. 4-11, the seal assembly 160 of the valve assembly 120 is configured to provide a seal around an outer surface of a surgical instrument (not shown) passing through the instrument valve housing 110 (FIG. 2).

The seal assembly 160 includes first, second, third, fourth, fifth, and sixth petals or sections 162, 164, 166, 168, 170, 172 movable from a first or unfolded configuration (FIG. 5) to folded configuration (FIG. 4). In the folded configuration, the seal assembly 160 forms a substantially planar, hexagonal member, with the first, second, third, fourth, fifth, and sixth sections 162, 164, 166, 168, 170, 172 of the seal assembly 160 defining an opening 161 therebetween to facilitate sealed passage of a surgical instrument (not shown) through the seal assembly 160. In examples, the opening 161 is 0,635 mm to 2,54 mm (0,025" to 0,100") (1 inch = 25,4 mm) in diameter. By forming the opening 161 out of the first, second, third, fourth, fifth, and sixth sections 162, 164, 166, 168, 170, 172 of the seal assembly 160 instead of as a continuous solid opening through as single seal member, the likelihood of the seal assembly 160 tearing during insertion, removal, and use of a surgical instrument therethrough is greatly reduced. Although shown including six (6) sections, it is envisioned that the seal assembly 160 may include as few as four (4) sections, and as many as eight (8) sections.

The first, second, third, fourth, fifth, and sixth sections 162, 164, 166, 168, 170, 172 of the seal assembly 160 are formed of an elastic material, e.g., rubber, polyisoprenes, or silicone elastomers. In examples, the first, second, third, fourth, fifth, and sixth sections 162, 164, 166, 168, 170, 172 may include one or more fabric layers.

With particular reference to FIG. 5, the first and second sections 162, 164 of the seal assembly 160, the second and third sections 164, 166, the third and fourth sections 166, 168, the fourth and fifth sections 168, 170, and the fifth and sixth section 170, 172 are connected to one another by a connector portion 162a, 164a, 166a, 168a, 170a, respectively. In examples, the connector portions 162a, 164a, 166a, 168a, 170a include a living hinge, or are otherwise formed to facilitate folding of the sections.

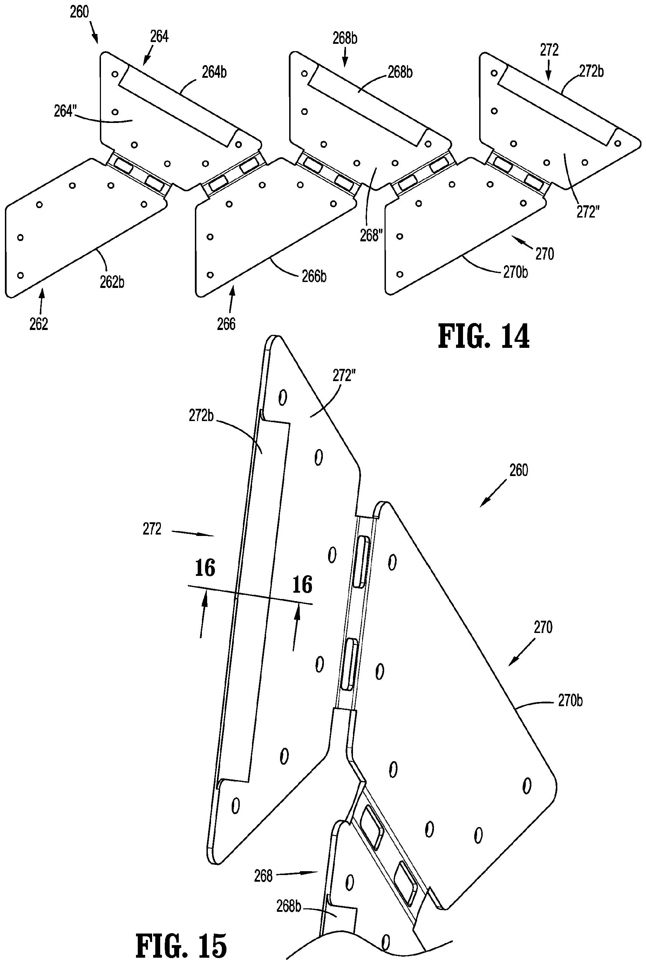

An inner edge 162b, 164b, 166b, 168b, 170b, 172b of the respective first, second, third, fourth, fifth, and sixth sections 162, 164, 166, 168, 170, 172 of the seal assembly 160 may be straight (FIG. 14), or may define a V-shape (FIG. 5). In examples, the V-shape defines an angle between one-hundred eighty degrees (180°) and two-hundred seventy-five degrees (275°). The V-shape of the inner edges 162b, 164b, 166b, 168b, 170b facilitates reception of a surgical instrument (not shown) through the seal assembly 160.

Each of the first, second, third, fourth, fifth, and sixth sections 162, 164, 166, 168, 170, 172 of the seal assembly 160 includes a wing-shaped body that is configured to partially overlap the respective connected second, third, fourth, fifth, and sixth sections 164, 166, 168, 170, 172 when the seal assembly 160 is in the folded configuration. The first, second, third, fourth, fifth, and sixth sections 162, 164, 166, 168, 170, 172 are also configured to partially overlap the respective adjacent third, fourth, fifth, sixth, first, and second sections 166, 168, 170, 172, 162, 164 and the respective adjacent sixth, first, second, third, fourth, and fifth sections 172, 162, 164, 166, 168. For example, the first section 162 overlaps the connected second section 164, and the adjacent third and sixth sections 166, 172. In this manner, a portion of each of the first, second, third, fourth, fifth, and sixth sections 162, 164, 166, 168, 170, 172 overlaps three sections.

Each of the first, second, third, fourth, fifth, and sixth sections 162, 164, 166, 168, 170, 172 defines a plurality of openings 163, 165, 167, 169, 171, 173 along an outer perimeter of each section 162, 164, 166, 168, 170, 172. In examples, and as shown, the plurality of openings 163, 165, 167, 169, 171, 173 is arranged such the first and last two openings of each plurality of openings 163, 165, 167, 169, 171, 173 align with the last and first two openings of the adjacent sections. For example, as noted above, the first section 162 overlaps the connected second section 164 and the adjacent third and sixth sections 166, 177. In this manner, the first two openings 163a of the plurality of openings 163 align with last two openings 167b of the plurality of openings 167 in the third section 166, and the second two openings 163b of the plurality of openings 163 in the first section 162 align with the first two openings 173a of the plurality of openings 173 of the sixth section when the seal assembly 160 is in the folded configuration.

The plurality of openings 163, 165, 167, 169, 171, 173 are configured to receive pins 186 (FIG. 3) of the retainer assembly 180 to maintain the seal assembly 160 in the folded condition and to secure the seal assembly 160 relative to the guard assembly 140 and the centering mechanism 130.

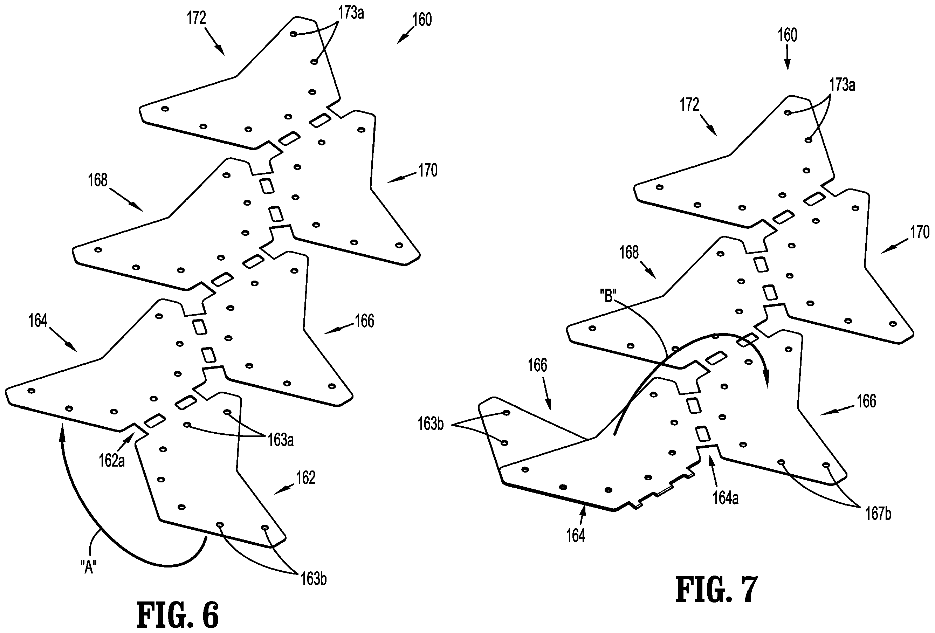

The method of folding the seal assembly 160 will now be described with reference to FIGS. 6-11. Referring initially to FIG. 6, the first section 162 of the seal assembly 160 is folded relative to the second section 164 at the hinge portion 162a between the first and second sections 162, 164, as indicated by arrow "A", such that a portion of the first section 162 adjacent the hinge portion 162a aligns with the portion of the second section 164 of the seal assembly 160 adjacent the hinge portion 162a. In this manner, the plurality of openings 163 in the portion of the first section 162 adjacent the hinge portion 162a aligns with the plurality of openings 165 in the overlapping portion of the second section 164 of the seal assembly 260 adjacent the hinge portions 162a.

Turning to FIG. 7, the second section 164 of the seal assembly 160 is folded relative to the third section 166 at the hinge portion 164a between the second and third sections 164, 166, as indicated by arrow "B", such that a portion of the second section 164 adjacent the hinge portion 164a overlaps the length of the portion of the third section 166 of the seal assembly 160 adjacent the hinge portion 164a. In this manner, the plurality of openings 165 in the portion of the second section 164 adjacent the hinge portion 164a aligns with the plurality of openings 167 in the overlapping portion of the third section 166 of the seal assembly 160 adjacent the hinge portions 164a.

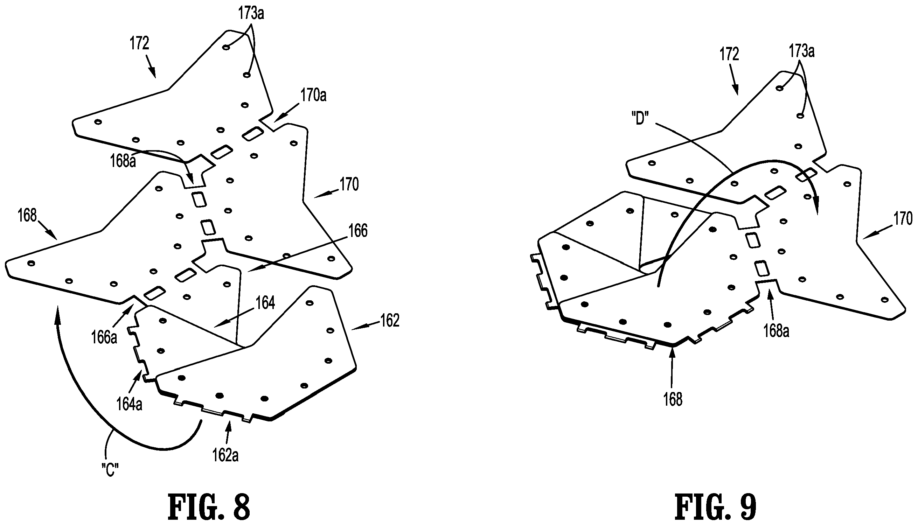

Referring to FIG. 8, the third section 166 of the seal assembly 160 is folded relative to the fourth section 168 of the seal assembly 160 at the hinge portion 166a between the third and fourth sections 166, 168, as indicated by arrow "C", such that the portion of the third section 166 adjacent the hinge portion 166a overlaps the portion of the fourth section 168 of the seal assembly 260 adjacent the hinge portion 166a. In this manner, the plurality of openings 167 in the portion of the third section 166 adjacent the hinge portion 166a aligns with the plurality of openings 169 in the overlapping portion of the fourth section 168 of the seal assembly 160 adjacent the hinge portions 166a.

With reference to FIG. 9, the fourth section 168 of the seal assembly 160 is folded relative to the fifth section 170 of the seal assembly 160 at the hinge portion 168a between the fourth and fifth sections 168, 170, as indicated by arrow "D", such that the portion of the fourth section 168 adjacent the hinge portion 168a overlaps the portion of the fifth section 170 of the seal assembly 160 adjacent the hinge portion 168a. In this manner, the plurality of openings 169 in the portion of the fourth section 168 adjacent the hinge portion 168a aligns with the plurality of openings 171 in the overlapping portion of the fifth section 170 adjacent the hinge portion 168a.

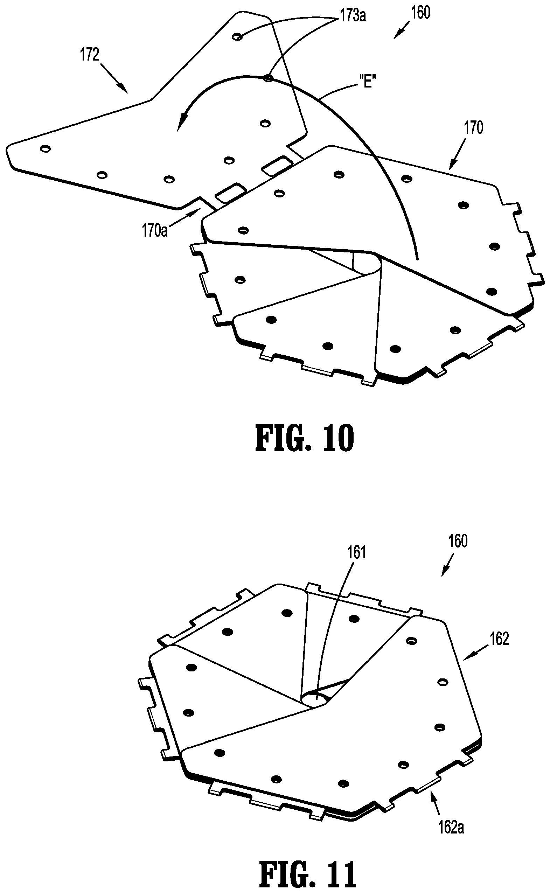

Turning to FIG. 10, the fifth section 170 of the seal assembly 160 is folded relative to the sixth section 172 at the hinge portion 170a between the fifth and sixth sections 170, 172, as indicated by arrow "E", such that the portion of the fifth section 170 adjacent the hinge portion 170a overlaps the portion of the sixth section 172 of the seal assembly 260 adjacent the hinge portion 170a. In this manner, the plurality of openings 171 in the portion of the fifth section 170 adjacent the hinge portion 170a aligns with the plurality of openings 173 in the overlapping portion of the sixth section 170 of the seal assembly 160 adjacent the hinge portion 170a.

FIG. 11 illustrates the seal assembly 160 in a fully folded configuration. In examples, a portion of the sixth section 172 of the seal assembly 160 is inserted under the first section 162 of the seal assembly 160 to interweave the first and sixth sections 162, 172. This interweaving increases the integrity of the seal assembly 160.

Referring back to FIGS. 2 and 3, the retainer assembly 180 of the valve assembly 120 is configured to secure the guard assembly 140 relative to the seal assembly 160, and secure the guard and seal assemblies 140, 160 to the centering mechanism 130. The retainer assembly 180 includes the upper retainer member 182, and a lower retainer member 184.

As noted above, the upper retainer member 182 includes a plurality of pins 186. The plurality of pins 186 extends from a bottom surface of the upper retainer member 182. Each pin of the plurality of pins 186 is configured to be lockingly received within an opening of a plurality of openings 185 (FIG. 3) of the lower retainer member 184. In examples, the plurality of pins 186 is welded, glued, adhered, bonded or otherwise secured within the plurality of openings 185 in the lower retainer member 184 to secure the upper retainer member 182 and the lower retainer member 184 together. Alternatively, the lower retainer member 184 may instead, or additionally, include a plurality of pins (not shown) with the upper retainer member 182 defining a plurality corresponding openings (not shown). Either or both of the upper and lower retainer members 182, 184 may include locking features (not shown) for engaging the plurality of pins and securing the upper retainer member 182 to the lower retainer member 184.

With particular reference to FIG. 2, the plurality of pins 186 of the upper retainer member 182 extends through the ring portion 142 of the guard assembly 140, through the seal assembly 160, through the inner annular ring 134 of the centering mechanism 130, and into the openings 185 in the lower retainer member 184.

During a surgical procedure utilizing access assembly 100, a surgical instrument (not shown) is introduced into the instrument valve housing 110 through the longitudinal passage 111 in the upper, lower, and inner housing sections 112, 114, 116. As described in the '377 and ` 120 Patents, the distal end of the surgical instrument engages the petals 144, 146, 148, 150 (FIG. 3) of the guard assembly 140 causing the respective petals 144, 146, 148, 150 to flex downward into contact with the seal assembly 160 to cause the central opening 163 of the seal assembly 160 to open to accommodate passage of the surgical instrument through the seal assembly 160. The guard assembly 130 minimizes damage to the seal assembly 160 during insertion of an instrument through the valve assembly 120. The guard assembly 130 operates to protect the seal assembly 160 from tearing or other damage as a surgical instrument is received through and withdrawn from the seal assembly 160. As discussed above, the multi-petal configuration of the seal assembly 160 reduces the likelihood of the seal assembly 160 tearing during insertion and/or removal of the surgical instrument therethrough.

With reference now to FIGS. 14-17, a seal assembly according to another example of the present disclosure is shown generally as seal assembly 260. The seal assembly 260 is substantially similar to the seal assembly 160 (FIGS. 4-11) described hereinabove, and will only be described in detail as relates to the differences therebetween.

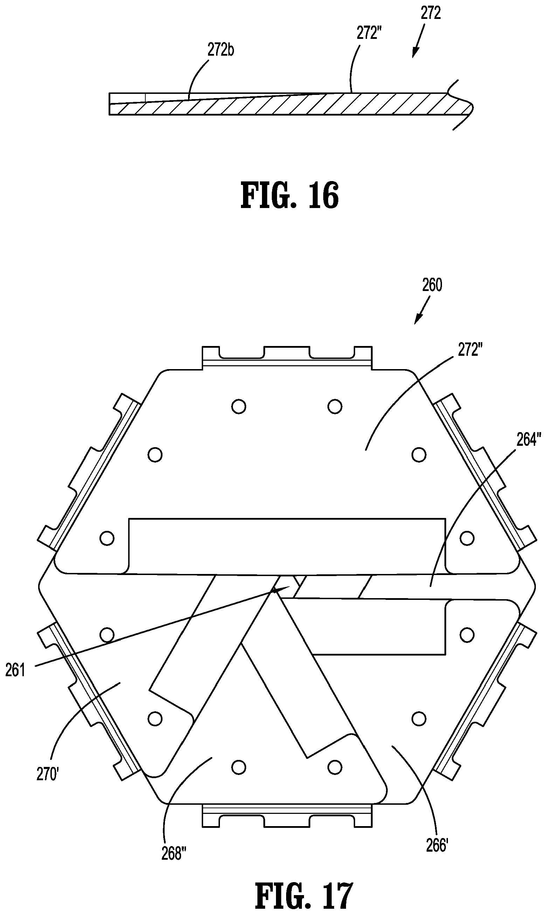

The seal assembly 260 is configured to provide a seal around an outer surface of a surgical instrument (not shown) passing through the instrument valve housing 110 (FIG. 2). The seal assembly 260 includes first, second, third, fourth, fifth, and sixth petals or sections 262, 264, 266, 268, 270, 272 foldable from a first or unfolded configuration (FIG. 14) to folded configuration (FIG. 17). In the folded configuration the seal assembly 260 forms a substantially planar, hexagonal member, with the first, second, third, fourth, fifth, and sixth sections 262, 264, 266, 268, 270, 272 of the seal assembly 260 defining an opening 261 therebetween to facilitate sealed passage of a surgical instrument (not shown) through the seal assembly 260.

Inner edges 262b, 264b, 266b, 268b, 270b, 272b of the respective first, second, third, fourth, fifth, and sixth sections 262, 264, 266, 268, 270, 272 of the seal assembly 160 are tapered. The tapered inner edges 262b, 266b, 270b of the first, third, and fifth sections 262, 266, 270, respectively, are disposed on first surfaces 262', 266', 270' (FIG. 17) of the respective first, third, and fifth sections 262, 266, 270, and the tapered inner edges 264b, 268b, 272b of the second, fourth, and sixth sections 264, 268, 274, respectively, are disposed on second surfaces 264", 268", 272" of the respective first, third, and fifth sections 262, 266, 270. The tapered inner edges 262b, 264b, 266b, 268b, 270b, 272b of the respective first, second, third, fourth, fifth, and sixth sections 262, 264, 266, 268, 270, 272 facilitate sealed receipt of a surgical instrument through the opening 261 in the seal assembly 260.

The seal assembly 260 is secured within the instrument valve housing 110 (FIG. 2) in a similar manner to seal assembly 160 (FIG. 3) described hereinabove. The seal assembly 260 operates in a similar manner to seal assembly 160.

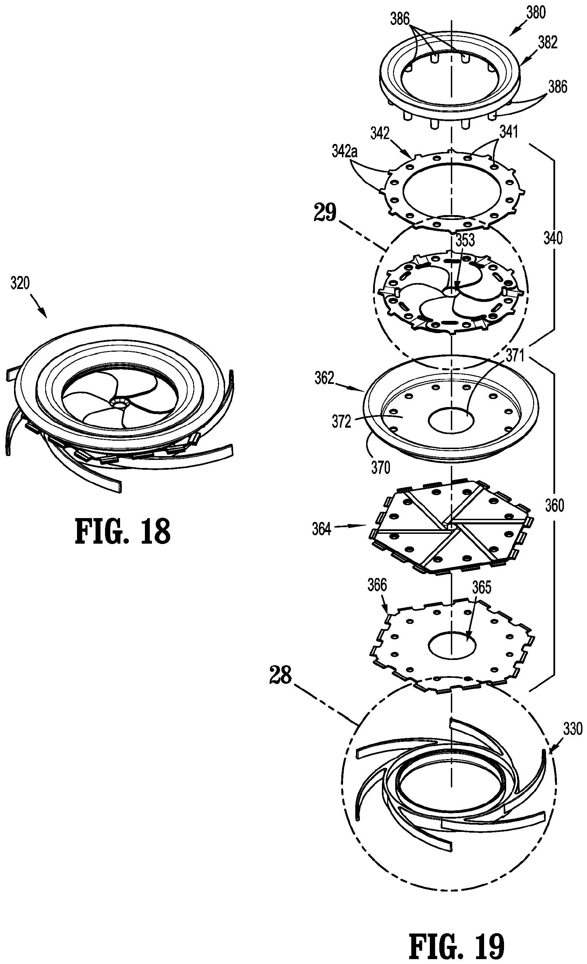

FIGS. 18 and 19 illustrate a valve assembly according to the invention shown generally as valve assembly 320. The valve assembly 320 is configured to be supported within an instrument valve housing, e.g., instrument valve housing 110 (FIG. 2), and includes a centering mechanism 330, a guard assembly 340, a seal assembly 360, and a retainer member 380. The centering mechanism 330 of the valve assembly 320 permits radial movement of the valve assembly 320 relative to the instrument valve housing 110 when a surgical instrument is received through the valve assembly 320, and returns the valve assembly 320 to a generally centered position once the surgical instrument is withdrawn from within the instrument valve housing 310. The guard assembly 340 protects the seal assembly 360 during insertion and withdrawal of a surgical instrument through the seal assembly 360. The seal assembly 360 provides sealed passage of the surgical instrument through the instrument valve housing 110. The retainer member 380 maintains the centering mechanism 330, the guard assembly 340, and the seal assembly 360 in an aligned relationship with one another.

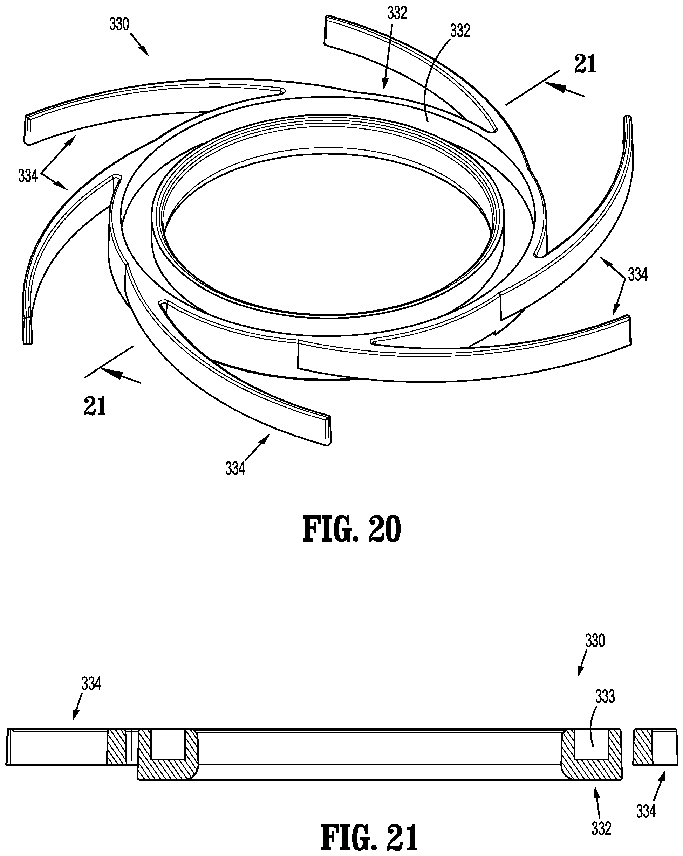

With reference to FIGS. 20 and 21, the centering mechanism 330 of the valve assembly 320 is configured to maintain the valve assembly 320 centered within the instrument valve housing 110 (FIG. 2). In examples, and as shown, the centering mechanism 330 includes an annular base 332 and a plurality of spokes 334 extending from the annular base 332. The annular base 332 defines a channel 333. The channel 333 is configured to receive a plurality of pins 386 extending from a retainer ring 382 of the retainer member 380. The plurality of pins 386 of the retainer member 380 may be frictionally secured within the annular base 332. Alternatively, the plurality of pins 386 may be secured within the channel 333 of the annular base with adhesive, welding, mechanical fasteners, or in any other suitable manner.

With continued reference to FIGS. 20 and 21, as described in U.S. Pat. App. Pub. No. 2015/0025477 ("the '477 publication"), the plurality of spokes 334 extending from the annular base 332 of the centering mechanism 330 acts as springs that bias the annular base 332 toward a centered position within the instrument valve housing 110. It is envisioned that the centering mechanism 330 may include multiple sets of spokes, as disclosed in the '477 publication.

Referring briefly back to FIG. 19, the guard assembly 340 of the valve assembly 320 includes a support ring 342 and a plurality of guard sections 344. Although shown having only a single support ring 342 that is disposed proximally of the plurality of guard sections 344, it is envisioned that the guard assembly may include support rings 342 disposed proximally and distally of the plurality of guard sections 344. The support ring 342 defines a plurality of openings 341 for receiving the plurality of pins 386 extending from the retainer ring 382 of the retainer member 380. The support ring 342 includes a plurality of radially outward extending tabs 342a. The plurality of tabs 342a facilitates positioning of the guard assembly 340 within the centering mechanism 330. As shown, the plurality of guard sections 344 includes six guard sections 346a-f. It is envisioned that the guard assembly 340 may include more or fewer than six guard sections 346a-f.

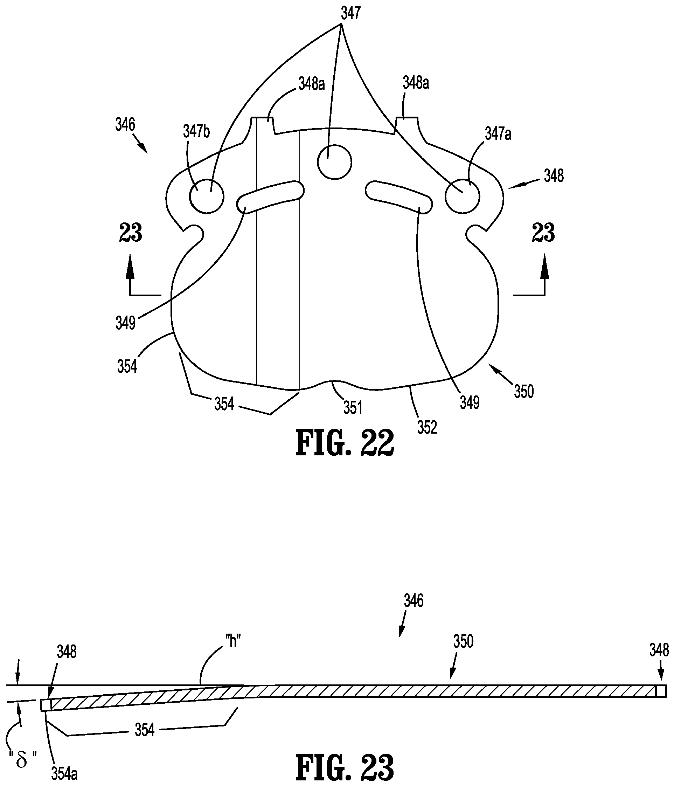

With reference to FIGS. 22 and 23, the six guard sections 346a-f are substantially similar, and will only be described in detail with regards to an exemplary guard section 346. The guard section 346 includes a base portion 348 and a guard portion 350 extending from the base portion 348. The base portion 348 of the guard section 346 defines a first opening 347a and a second opening 347b, forming a plurality of openings 347, for receiving the plurality of pins 386 of the retainer member 380 (FIG. 19). Although shown having three openings 347, it is envisioned that the guard sections 346 may include any number of openings corresponding in number and placement to the plurality of pins 386 of the retainer member 380. At least one tab 348a extends radially outward from the base portion 348. As shown, the guard section 346 includes a pair of tabs 348a. The tabs 348a facilitate positioning of the guard assembly 340 within the centering mechanism 330.

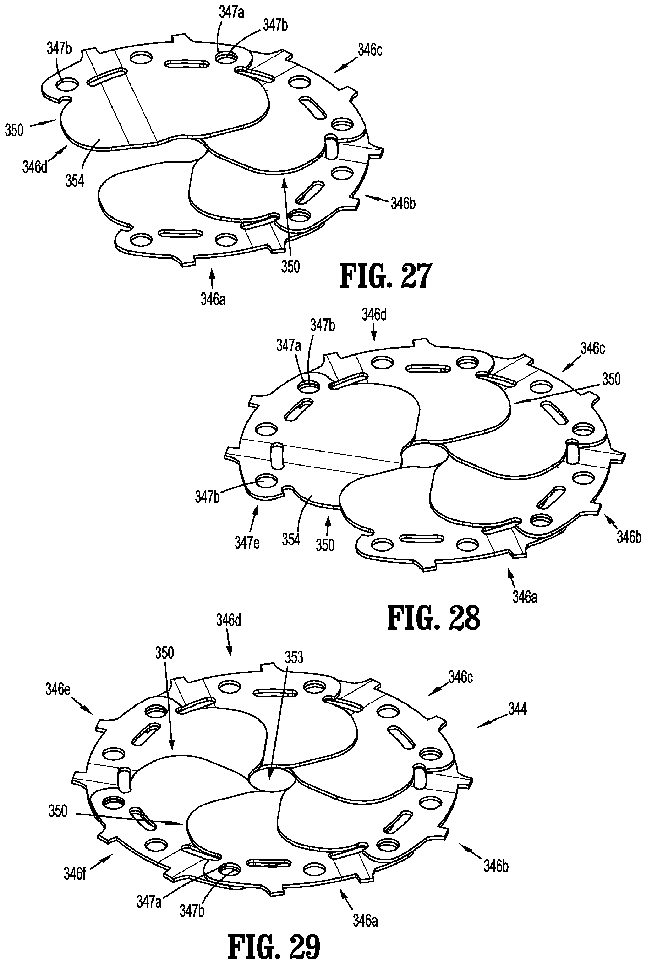

The guard section 346 of the guard assembly 340 defines at least one longitudinal slot 349 disposed between the base portion 348 and the guard portion 350. As shown, the guard section 346 defines a pair of longitudinal slots 349. The longitudinal slots 349 facilitate flexing of the guard portion 350 relative to the base portion 348. The guard portion 350 of the guard section 346 defines a substantial kidney-shape including an indent 351 along an inner edge 352. When the plurality of guard sections 346a-f is assembled (FIG. 29), the indents 351 along the inner edge 352 of the guard portion 350 form an opening 353 (FIG. 29) for accommodating a surgical instrument (not shown).

With reference to FIG. 23, a first side 354 of the guard section 346 angles downward in relation to the remainder of the guard section 346. An edge 354a of the first side 354 of the guard section 346 is spaced a distance "δ" from a horizontal plane "h" defined by a surface of the remainder of the guard section 346. The downward angling of the first side 354 of the guard section 346 accommodates the overlapping portions of the adjacent guard sections 346a-f when the plurality of guard sections 344 of the guard assembly 340 is assembled (FIG. 29) such that the plurality of guard sections 344 lays in a substantially planar arrangement.

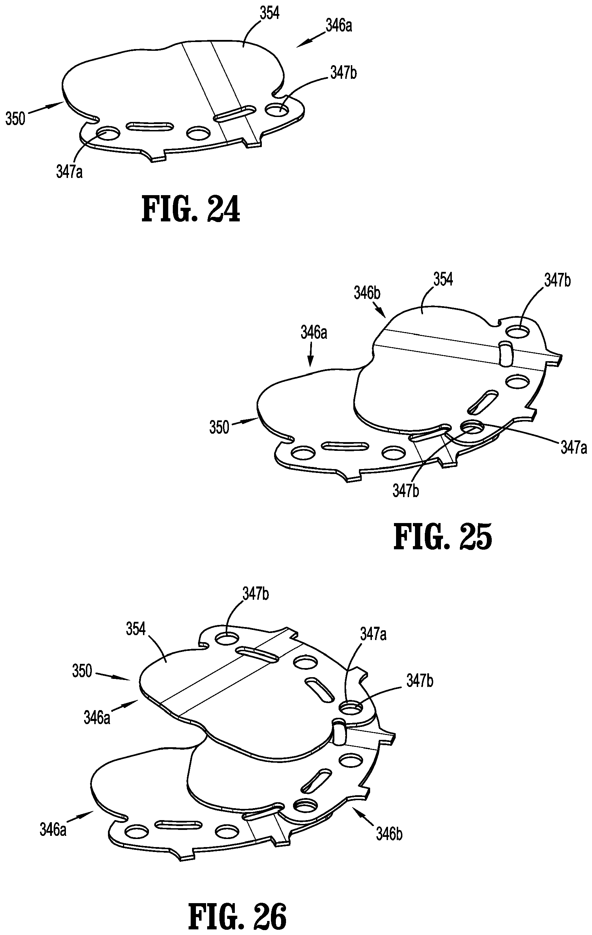

With reference now to FIGS. 24-29, the method of assembling the plurality of guard sections 344 will be described. Referring initially to FIG. 24, the first guard section 346a is positioned such that the first side 354 of the guard portion 350 of the first guard section 346a angles downward.

Turning to FIG. 25, the second guard section 346b is positioned such that the guard portion 350 of the second guard section 346b overlaps the first side 354 of the first guard section 346a with the first side 354 of the guard portion 350 of the second guard section 346b angling downward and away from the first guard section 346a. As such, a first opening 347a of the second guard section 346b overlaps a second opening 347b of the first guard section 346a.

Referring to FIG. 26, the third guard section 346c is positioned such that the guard portion 350 of the third guard section 346c overlaps the first side 354 (FIG. 25) of the second guard section 346a with the first side 354 of the guard portion 350 of the third guard section 346c angling downward and away from the second guard section 346b. As such, a first opening 347a of the third guard section 346c overlaps a second opening 347b of the second guard section 346b.

With reference to FIGS. 27-29, the fourth, fifth, and sixth guard sections 346d, 346e, 346f, are positioned similarly, with the exception that the first side (not shown) of the guard portion 350 of the sixth guard section 346f is positioned under the guard portion 350 of the first guard section 346a to complete the assembly of the plurality of guard sections 344 of the guard assembly 340.

As described in further detail below, the plurality of guard sections 344 of the guard assembly 340 are maintained in the assembled configuration by retainer assembly 380.

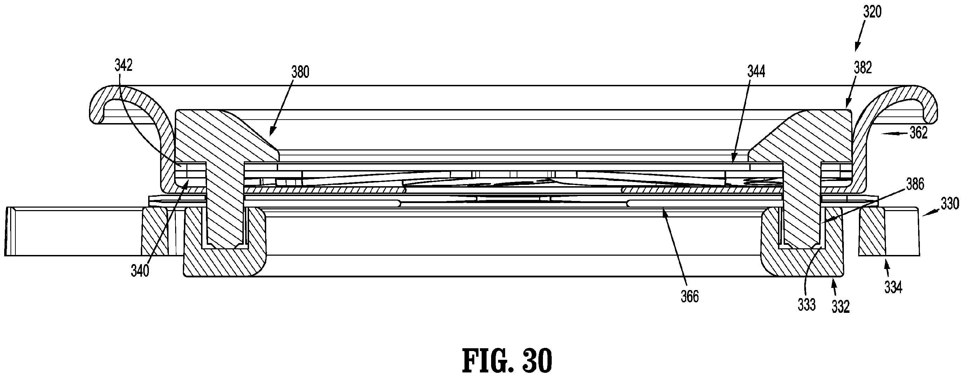

With reference back to FIG. 19, and additional reference to FIG. 30, the seal assembly 360 of the valve assembly 320 includes a proximal seal member 362, an intermediate seal assembly 364, and a distal seal member 368. The proximal seal member 362 includes an annular flange 370 and a seal portion 372 supported within the annular flange 370. The seal portion 372 is formed of an elastic material, e.g., rubber, and defines a central opening 371. The annular flange 370 is configured to direct a surgical instrument (not shown) through the central opening 371 in the seal portion 372. The intermediate seal assembly 364 is substantially similar to the seal assemblies 160, 260 described hereinabove. The distal seal member 366 corresponds in shape to the intermediate seal assembly 364. The distal seal member 366 is formed of an elastic material, e.g., rubber, and defines a central opening 365.

With particular reference to FIG. 30, the proximal seal member 362 and the distal seal member 366 of the seal assembly 370 operate together to provide support for the intermediate seal assembly 364. More particularly, the intermediate seal assembly 364 is sandwiched between the seal portion 372 of the proximal seal member 362 and the distal seal member 366. As a surgical instrument (not shown) is received through the seal assembly 360, the distal seal member 366 supports the intermediate seal assembly 364. Similarly, as the surgical instrument is withdrawn from the seal assembly 360, the proximal seal member 362 supports the intermediate seal assembly 364.

Although shown and described with reference to the seal assembly 360, it is envisioned that the valve assembly 320 may include any suitable seal assembly.

As noted above, the retainer ring 382 of the retainer member 380 includes a plurality of pins 386 that is received through the plurality of openings 342, 347 in the support ring 342 and the plurality of guard sections 344 of the guard assembly 340, respectively, and through the seal assembly 360. The plurality of retaining pins 386 is secured within the channel 333 of the annular base 332 of the centering mechanism 330 to maintain the guard assembly 340 and the seal assembly 360 in an assembled configuration.

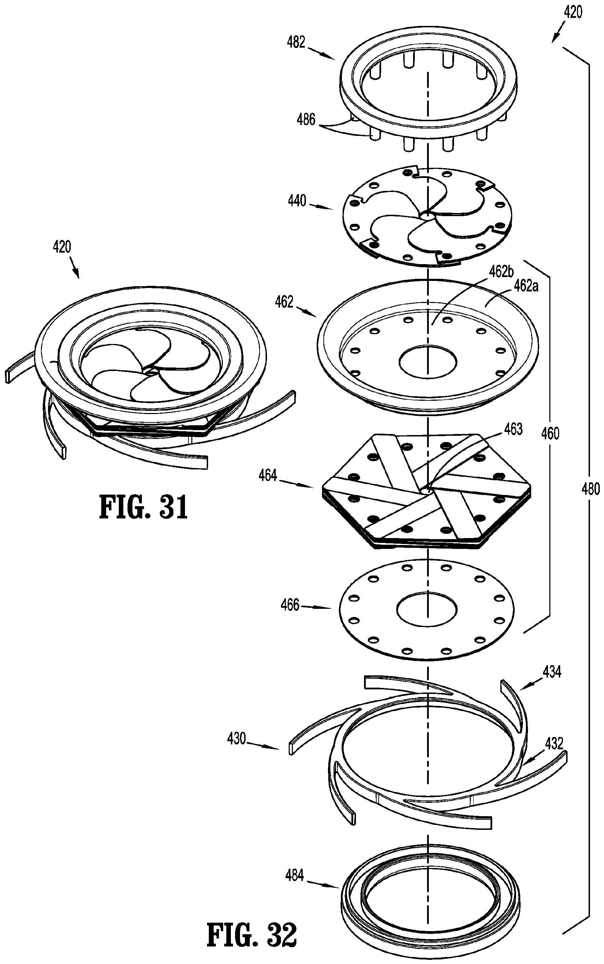

FIGS. 31 and 32 illustrate a valve assembly according to another example of the disclosure shown generally as valve assembly 420. The valve assembly 420 is configured to be supported within an instrument valve housing, e.g., instrument valve housing 110 (FIG. 2), and includes a centering mechanism 430, a guard assembly 440, a seal assembly 460, and a retainer assembly 480.

The centering mechanism 430 of the valve assembly 420 permits radial movement of the valve assembly 420 relative to the instrument valve housing 110 (FIG. 2) when a surgical instrument is received through the valve assembly 420, and returns the valve assembly 420 toward a generally centered position once the surgical instrument is withdrawn from within the instrument valve housing 410. As shown, the centering mechanism 430 is substantially similar to centering mechanism 330 described hereinabove. It is envisioned that valve assembly 420 may include alternative centering mechanisms including, for example, the centering mechanism 130 described hereinabove.

The guard assembly 440 of the valve assembly 420 protects the seal assembly 460 during insertion and withdrawal of a surgical instrument through the seal assembly 460. As shown, the guard assembly 440 is substantially similar to the guard assembly 440 described hereinabove. It is envisioned that the valve assembly 420 may include alternative guard assemblies including, for example, guard assembly 140 described hereinabove. The seal assembly 460 provides sealed passage of the surgical instrument through the instrument valve housing 110. The retainer member 480 maintains the centering mechanism 430, the guard assembly 440, and the seal assembly 460 in an aligned relationship with one another.

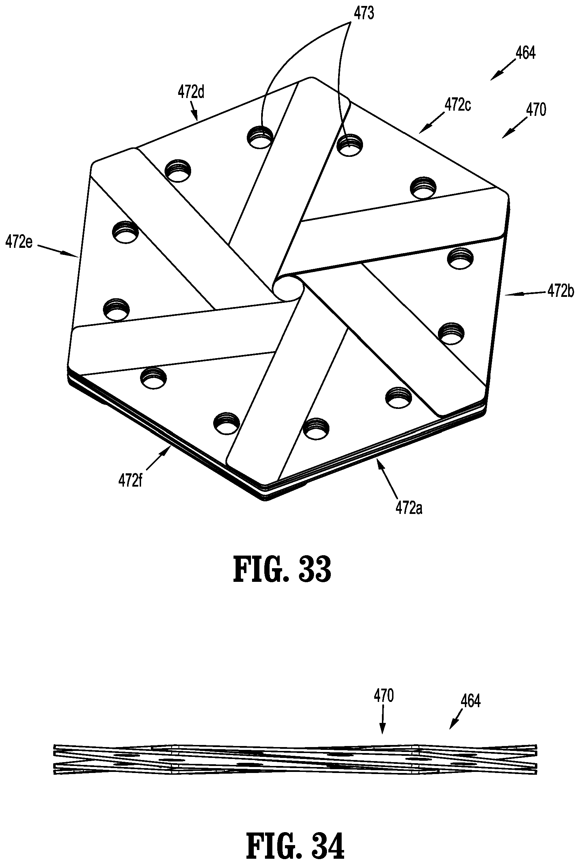

With reference continued reference to FIGS. 31 and 32, and additional reference to FIGS. 33 and 34, the seal assembly 460 of the valve assembly 420 includes a proximal seal member 462, an intermediate seal assembly 464, and a distal seal member 466. The proximal seal member 462 is substantially similar to proximal seal member 362 described hereinabove, and includes an annular flange 462a and a seal portion 462b supported within the annular flange 462a. The intermediate seal assembly 464 is similar to the seal assemblies 160, 260 described hereinabove, and will be described in further detail below. The distal seal member 366 corresponds in size and shape to the seal portion 462b of the proximal seal member 462. The proximal seal member 462 and the distal seal member 466 of the seal assembly 470 operate together to provide support for the intermediate seal assembly 464.

The intermediate seal assembly 464 of the seal assembly 460 includes a plurality of seal sections 470 (first, second, third, fourth, fifth, and sixth seal sections 472a-f) arranged in an overlapping or stacked configuration. In the overlapping configuration, the intermediate seal assembly 464 forms a substantially planar, hexagonal member, with the first, second, third, fourth, fifth, and sixth seal sections 472a-f of the seal assembly 464 defining an opening 463 (FIG. 32) to facilitate sealed passage of a surgical instrument (not shown) through the seal assembly 460. Although the seal sections 472a-f of the seal assembly 464 are shown and described as forming separate components, it is envisioned that the seal sections may be integrally formed with, or otherwise secured to, a central portion. In this manner, the seal sections are folded over the central portion to form the overlapping or interwoven configuration.

In examples, the opening 463 of the intermediate seal assembly 464 is 0.025" to 0.100" in diameter. By forming the opening 463 out of the first, second, third, fourth, fifth, and sixth seal sections 472a-f of the seal assembly 460 instead of as a continuous solid opening through a single seal member, the likelihood of the seal assembly 460 tearing during insertion, removal, and manipulation of a surgical instrument therethrough is greatly reduced. Although shown including six (6) seal sections, it is envisioned that the seal assembly 460 may include as few as four (4) seal sections, and as many as eight (8) seal sections.

The first, second, third, fourth, fifth, and sixth seal sections 472a-f of the seal assembly 460 are formed of an elastic material, e.g., rubber, polyisoprenes, or silicone elastomers. In examples, the first, second, third, fourth, fifth, and sixth seal sections 472a-f may include one or more fabric layers.

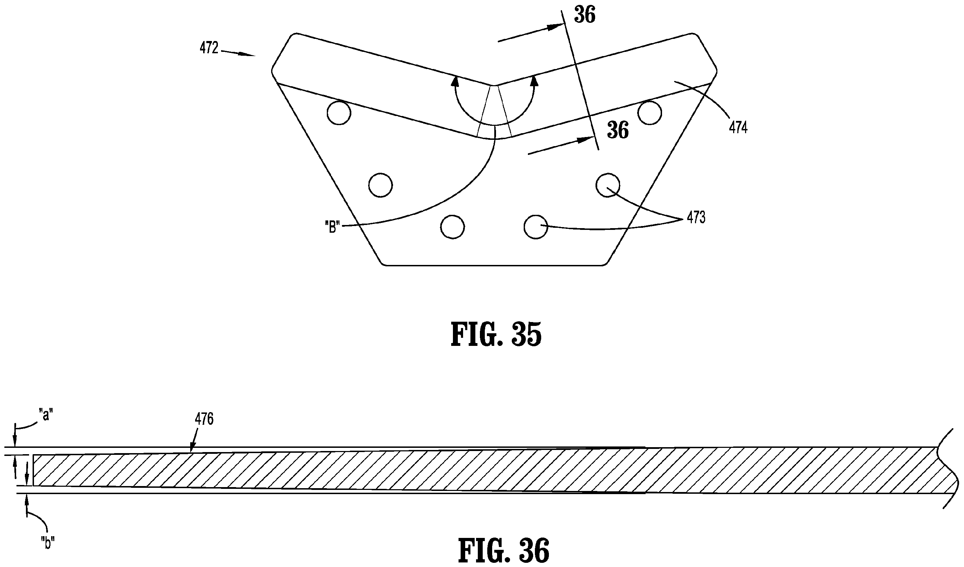

With reference now to FIGS. 35 and 36 the first, second, third, fourth, fifth, and sixth seal sections 472a-f are substantially identical and will only be described in detail with regards to an exemplary seal section 472. The exemplary seal section 472 is substantially similar to the seal sections of the seal assemblies 160, 260 described hereinabove. More particularly, the seal section 472 includes a wing-shaped body configured to partially overlap adjacent seal sections. An inner or edge portion 476 of the seal section 472 may be straight (FIG. 14), or may define a V-shape, as shown. In examples, the V-shape defines an angle "β" between about one-hundred eighty degrees (180°) and about two-hundred seventy-five degrees (275°). The V-shape of the inner portion 474 facilitates reception of a surgical instrument (not shown) through the seal assembly 460.

As shown in FIG. 36, the inner portion 474 of the seal section 472 is tapered, e.g., the thickness of the inner portion 474 decreases from proximal and distal surfaces a distance "a", "b" respectively. Although shown with a symmetric taper, e.g., distances "a", "b" are the same, it is envisioned that the inner portion 474 of the seal section 472 may be tapered on only one of the proximal and distal surface, or that the distances "a", "b" may be different. The tapered configuration of the inner portion 474 facilitates insertion and withdrawal of a surgical instrument through the seal assembly 460. The tapered configuration of the inner portion 474 of the seal sections 472 may also provide improved sealing about a surgical instrument received through the seal assembly 470.

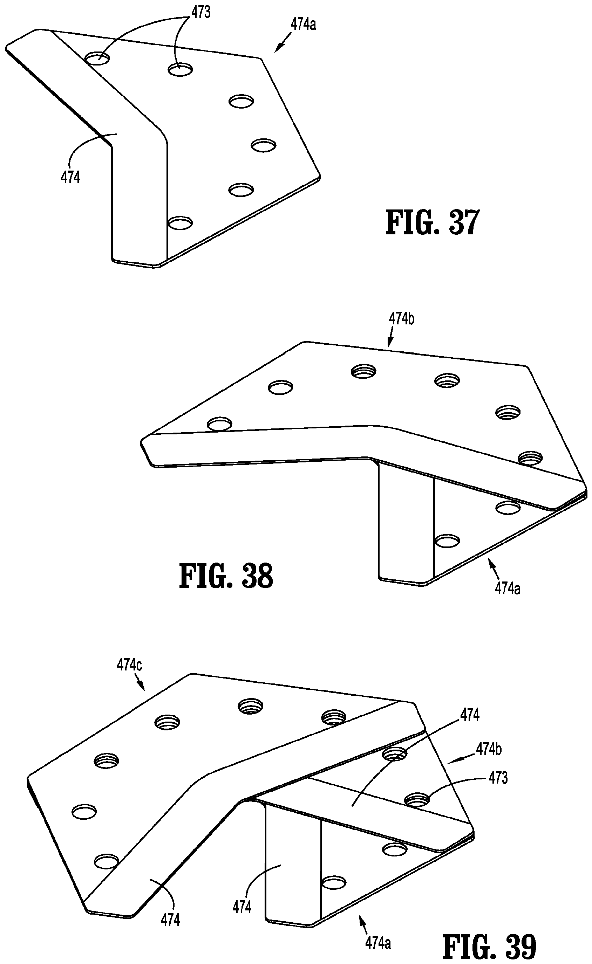

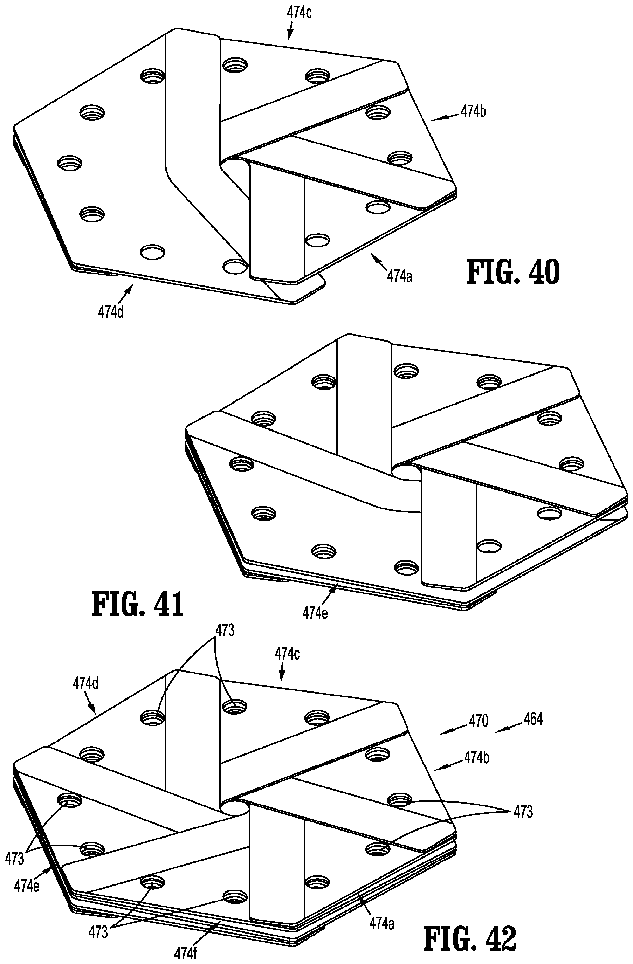

FIGS. 37-42 illustrate the assembly of the intermediate seal assembly 464. More particularly, the figures illustrate the stacked and overlapping configuration of the plurality of seal sections 470. The plurality of seal sections 470 of the intermediate seal assembly 464 are maintained in the assembled configuration by retainer assembly 480 (FIG. 32). More particularly, a plurality of pins 486 (FIG. 32) of an upper retainer member 482 of the retainer assembly 480 is received through a plurality of openings 473 in the seal sections 472. The retainer assembly 480 further maintains the seal assembly 460 relative to the centering mechanism 430, and the guard assembly 440. The plurality of pins 486 of the retainer assembly 480 are secured to a lower retainer member 484 (FIG. 32) of the retainer assembly 480. More particularly, the plurality of pins 486 is secured within a channel 485 of the lower retainer member 484 in any suitable fashion, including, for example, by friction fit, welding, or adhesive.

While various embodiments of the present disclosure have been shown and described herein, it will be obvious to those skilled in the art that these embodiments are provided by way of example only. Numerous variations, changes, and substitutions will now occur to those skilled in the art without departing from the present disclosure. Accordingly, it is intended that the invention be limited only by the scope of the appended claims.

## Claims

1. A surgical access assembly (100) comprising:
an instrument valve housing (110) defining a cavity; and
a valve assembly (320) disposed within the cavity of the instrument valve housing, the valve assembly including:
a guard assembly (340) including a support ring (342) and a plurality of guard sections (346) disposed distally of the support ring;
a seal assembly (360) disposed adjacent to the guard assembly (340), the seal assembly including a proximal seal member (362), an intermediate seal assembly (364), and a distal seal member (366); and
a centering mechanism (330) for maintaining the seal assembly (360) and the guard assembly (340) centered within the cavity of the instrument valve housing (110),
**characterised in that** each guard section (346) of the plurality of guard sections includes a first side (354) that is angled downwardly in relation to the remainder of the guard section to accommodate an overlapping portion of an adjacent guard section of the plurality of guard sections, whereby the plurality of guard sections lays in a substantially planar arrangement when the valve assembly (320) is in an assembled condition.

2. The access assembly of claim 1, wherein guard assembly (340) includes six guard sections (346).

3. The access assembly of any preceding claim, wherein each guard section (346) of the plurality of guard sections includes a guard portion (350) having a substantially kidney shape.

4. The access assembly of claim 3, wherein each guard portion (350) includes an indentation (351) and is configured such that when the plurality of guard sections (346) are assembled, the indentations form an opening (353).

5. The access assembly of any preceding claim, wherein at least one of the support ring (342) and the plurality of guard sections (346) include a plurality of outward extending tabs (348a) for aligning the guard assembly (340) with the centering mechanism (330) when the valve assembly (320) is in an assembled configuration.

6. The access assembly of any preceding claim, wherein the intermediate seal assembly (364) includes a plurality of seal sections (162, 262, 462) in a stacked configuration.

7. The access assembly of claim 6, wherein the intermediate seal assembly (364) includes six seal sections.

8. The access assembly of claim 6 or claim 7, wherein each seal section of the plurality of seal sections (162, 462) includes a wing shape.

9. The access assembly of any of claims 6 to 8, wherein an inner edge (262b) of each seal section (262) of the plurality of seal sections is straight.

10. The access assembly of any of claims 6 to 9, wherein at least one of a top surface (270') and a bottom surface (270") of an inner portion of each seal section (262, 462) tapers.

11. The access assembly of any of claims 6 to 10, wherein the intermediate seal assembly (364) is hexagonal.

12. The access assembly of any preceding claim, wherein the proximal seal member (362) includes an annular flange (370) and a seal portion (372) supported within the annular flange.

13. The access assembly of claim 12, wherein the proximal and distal seal members (362, 366) operate to support the intermediate seal assembly (364).

14. The access assembly of claim 13, further including a retainer assembly (180, 480) including upper and lower retainer members (182, 482, 184, 484), wherein at least one of the upper and lower retainer members includes a plurality of pins (186, 486) receivable through the guard assembly and seal assembly for retaining the guard and seal assemblies relative to each other.

15. The access assembly of claim 13, further including a retaining member (380) having a plurality of pins (386), and wherein the centering mechanism (330) includes an annular base (332) defining a channel (333) for receiving the plurality of pins for maintaining the valve assembly (320) in an assembled configuration.

## Patentansprüche

1. Chirurgische Zugangsanordnung (100), umfassend:
ein Instrumentenventilgehäuse (110), in dem ein Hohlraum definiert ist, und
eine Ventilanordnung (320), die in dem Hohlraum des Instrumentenventilgehäuses angeordnet ist, wobei die Ventilanordnung Folgendes aufweist:
eine Schutzanordnung (340), die einen Stützring (342) und eine Vielzahl von distal von dem Stützring angeordneten Schutzsektionen (346) aufweist,
eine Dichtungsanordnung (360), die der Schutzanordnung (340) benachbart angeordnet ist, wobei die Dichtungsanordnung ein proximales Dichtungsglied (362), eine Zwischendichtungsanordnung (364) und ein distales Dichtungsglied (366) aufweist, und
einen Zentrierungsmechanismus (330), um die Dichtungsanordnung (360) und die Schutzanordnung (340) in dem Hohlraum des Instrumentenventilgehäuses (110) zentriert zu halten,
**dadurch gekennzeichnet, dass** jede Schutzsektion (346) der Vielzahl von Schutzsektionen eine erste Seite (354) aufweist, die bezüglich des Rests der Schutzsektion nach unten abgewinkelt ist, um einen überlappenden Abschnitt einer benachbarten Schutzsektion der Vielzahl von Schutzsektionen unterzubringen, wobei die Vielzahl von Schutzsektionen in einer im Wesentlichen planaren Anordnung liegen, wenn die Ventilanordnung (320) in einem zusammengebauten Zustand ist.

2. Zugangsanordnung nach Anspruch 1, wobei die Schutzanordnung (340) sechs Schutzsektionen (346) aufweist.

3. Zugangsanordnung nach einem der vorhergehenden Ansprüche, wobei jede Schutzsektion (346) der Vielzahl von Schutzsektionen einen Schutzabschnitt (350) aufweist, der im Wesentlichen nierenförmig ist.

4. Zugangsanordnung nach Anspruch 3, wobei jeder Schutzabschnitt (350) eine Vertiefung (351) aufweist und so ausgestaltet ist, dass die Vertiefungen (353) eine Öffnung (353) bilden, wenn die Vielzahl von Schutzsektionen (346) zusammengebaut sind.

5. Zugangsanordnung nach einem der vorhergehenden Ansprüche, wobei der Stützring (342) und/oder die Vielzahl von Schutzsektionen (346) eine Vielzahl von sich nach außen erstreckenden Laschen (348a) zum Ausrichten der Schutzanordnung (340) auf den Zentrierungsmechanismus (330), wenn die Ventilanordnung (320) in einer zusammengebauten Konfiguration ist, aufweisen.

6. Zugangsanordnung nach einem der vorhergehenden Ansprüche, wobei die Zwischendichtungsanordnung (364) eine Vielzahl von Dichtungssektionen (162, 262, 462) in einer Stapelkonfiguration aufweist.

7. Zugangsanordnung nach Anspruch 6, wobei die Zwischendichtungsanordnung (364) sechs Dichtungssektionen aufweist.

8. Zugangsanordnung nach Anspruch 6 oder Anspruch 7, wobei jede Dichtungssektion der Vielzahl von Dichtungssektionen (162, 462) Flügelform aufweist.

9. Zugangsanordnung nach einem der Ansprüche 6 bis 8, wobei ein Innenrand (262b) jeder Dichtungssektion (262) der Vielzahl von Dichtungssektionen gerade ist.

10. Zugangsanordnung nach einem der Ansprüche 6 bis 9, wobei sich eine obere Fläche (270') und/oder eine untere Fläche (270") eines inneren Abschnitts jeder Dichtungssektion (262, 462) verjüngt.

11. Zugangsanordnung nach einem der Ansprüche 6 bis 10, wobei die Zwischendichtungsanordnung (364) hexagonal ist.

12. Zugangsanordnung nach einem der vorhergehenden Ansprüche, wobei das proximale Dichtungsglied (362) einen ringförmigen Flansch (370) und einen Dichtungsabschnitt (372) aufweist, der in dem ringförmigen Flansch gestützt ist.

13. Zugangsanordnung nach Anspruch 12, wobei das proximale und das distale Dichtungsglied (362, 366) zum Stützen der Zwischendichtungsanordnung (364) wirken.

14. Zugangsanordnung nach Anspruch 13, ferner aufweisend eine Halteranordnung (180, 480), die ein oberes und ein unteres Halterglied (182, 482, 184, 484) aufweist, wobei das obere und/oder das untere Halterglied eine Vielzahl von Stiften (186, 486) aufweisen, die durch die Schutzanordnung und die Dichtungsanordnung aufnehmbar sind, um die Schutz- und die Dichtungsanordnung in Beziehung zueinander zu halten.

15. Zugangsanordnung nach Anspruch 13, ferner aufweisend ein Halteglied (380) mit einer Vielzahl von Stiften (386) und wobei der Zentrierungsmechanismus (330) eine ringförmige Basis (332) aufweist, die einen Kanal (333) zur Aufnahme der Vielzahl von Stiften definiert, um die Ventilanordnung (320) in einer zusammengebauten Konfiguration zu halten.

## Revendications

1. Ensemble d'accès chirurgical (100) comprenant :
un boîtier de valve d'instrument (110) définissant une cavité ; et
un ensemble de valve (320) disposé à l'intérieur de la cavité du boîtier de valve d'instrument, l'ensemble de valve incluant :
un ensemble de garde (340) comprenant une bague de support (342) et une pluralité de sections de garde (346) disposées de manière distale par rapport à la bague de support ;
un ensemble joint d'étanchéité (360) disposé de manière adjacente à l'ensemble de garde (340), l'ensemble joint d'étanchéité comprenant un élément de joint d'étanchéité proximal (362), un ensemble joint d'étanchéité intermédiaire (364) et un élément de joint d'étanchéité distal (366), et
un mécanisme de centrage (330) pour maintenir l'ensemble joint d'étanchéité (360) et l'ensemble de garde (340) centrés à l'intérieur de la cavité du boîtier de valve d'instrument (110),
**caractérisé en ce que** chaque section de garde (346) de la pluralité de sections de garde comprend un premier côté (354) qui est incliné vers le bas par rapport au reste de la section de garde pour recevoir une partie de chevauchement d'une section de garde adjacente de la pluralité de sections de garde, la pluralité de sections de garde reposant ainsi dans un agencement sensiblement plan lorsque l'ensemble de valve (320) est dans une condition assemblée.

2. Ensemble d'accès selon la revendication 1, l'ensemble de garde (340) comprenant six sections de garde (346).

3. Ensemble d'accès selon n'importe quelle revendication précédente, chaque section de garde (346) de la pluralité de sections de garde comprenant une partie de garde (350) ayant une forme sensiblement de rein.

4. Ensemble d'accès selon la revendication 3, chaque partie de garde (350) comprenant une indentation (351) et étant configurée de telle sorte que lorsque la pluralité de sections de garde (346) est assemblée, les indentations forment une ouverture (353).

5. Ensemble d'accès selon n'importe quelle revendication précédente, au moins l'une de la bague de support (342) et de la pluralité de sections de garde (346) comprenant une pluralité de pattes s'étendant vers l'extérieur (348a) pour aligner l'ensemble de garde (340) avec le mécanisme de centrage (330) lorsque l'ensemble de valve (320) est dans une configuration assemblée.

6. Ensemble d'accès selon n'importe quelle revendication précédente, l'ensemble joint d'étanchéité intermédiaire (364) comprenant une pluralité de sections de joint d'étanchéité (162, 262, 462) dans une configuration empilée.

7. Ensemble d'accès selon la revendication 6, l'ensemble joint d'étanchéité intermédiaire (364) comprenant six sections de joint d'étanchéité.

8. Ensemble d'accès selon la revendication 6 ou selon la revendication 7, chaque section de joint d'étanchéité de la pluralité de sections de joint d'étanchéité (162, 462) comprenant une forme d'aile.

9. Ensemble d'accès selon l'une quelconque des revendications 6 à 8, un bord intérieur (262b) de chaque section de joint d'étanchéité (262) de la pluralité de sections de joint d'étanchéité étant droit.

10. Ensemble d'accès selon l'une quelconque des revendications 6 à 9, au moins l'une d'une surface supérieure (270') et d'une surface inférieure (270") d'une partie intérieure de chaque section de joint d'étanchéité (262, 462) étant effilée.

11. Ensemble d'accès selon l'une quelconque des revendications 6 à 10, l'ensemble joint d'étanchéité intermédiaire (364) étant hexagonal.

12. Ensemble d'accès selon n'importe quelle revendication précédente, l'élément de joint d'étanchéité proximal (362) comprenant une bride annulaire (370) et une partie d'étanchéité (372) supportée à l'intérieur de la bride annulaire.

13. Ensemble d'accès selon la revendication 12, les éléments de joint d'étanchéité proximal et distal (362, 366) fonctionnant pour supporter l'ensemble joint d'étanchéité intermédiaire (364).

14. Ensemble d'accès selon la revendication 13, comprenant en outre un ensemble de retenue (180, 480) comprenant des éléments de retenue supérieur et inférieur (182, 482, 184, 484), au moins l'un des éléments de retenue supérieur et inférieur comprenant une pluralité de broches (186, 486) pouvant être reçues à travers l'ensemble de garde et l'ensemble joint d'étanchéité pour retenir les ensembles de garde et de joint l'un par rapport à l'autre.

15. Ensemble d'accès selon la revendication 13, comprenant en outre un élément de retenue (380) ayant une pluralité de broches (386), et le mécanisme de centrage (330) comprenant une base annulaire (332) définissant un canal (333) pour recevoir la pluralité de broches pour maintenir l'ensemble de valve (320) dans une configuration assemblée.
